# NEW EUROPEAN PATENT SPECIFICATION

(11) **EP 0 371 497 B2**
(45) Date of publication and mention of the opposition decision: **02.10.1996**
(45) Mention of the grant of the patent: 27.04.1994
(21) Application number: 89122072.5
(22) Date of filing: 29.11.1989
(51) Int. Cl.: A61M 25/00

(54) **Urethra catheter and manufacturing method therefor**
Urethralkatheter und dessen Herstellungsverfahren
Cathéter urétral et procédé pour sa fabrication

(30) Priority: 29.11.1988 JP 299685/88; 27.10.1989 JP 278541/89
(43) Date of publication of application: 06.06.1990
(73) Proprietor: TERUMO KABUSHIKI KAISHA, Tokyo 151 (JP)
(72) Inventor: Takeoka, Norio c/o Terumo K. K., Fujinomiya-shi Shizuoka (JP); Sugisawa, Yasuhiko c/o Terumo K. K., Fujinomiya-shi Shizuoka (JP)
(74) Representative: Casalonga, Axel

(56) References cited:
- FR-A- 2 371 933
- GB-A- 1 428 766

## Description

The present invention relates to a urethra catheter disposed from the urethra to the urinary bladder and capable of conducting continuous urination and a manufacturing method therefor.

In general, urethra catheters are arranged to include: a shaft tube having a main lumen and a sub-lumen and further having a urine inlet port at the front portion thereof, the urine inlet port being connected to the main lumen; a balloon fastened on the outer surface of the front portion of the shaft tube to cover an opening portion of an air lumen forced in the shaft tube; and a base portion disposed in the base end portion of the shaft tube, having a urine outlet port connected to the main lumen, and further having a fluid inlet/outlet port connected to the sub-lumen and capable of letting in or letting out fluid for expanding the balloon.

Hitherto, a urethra catheter is arranged such that each of the above-described shaft tube, the above-described balloon, and the above-described base portion is made of latex rubber or silicone rubber.

However, the above-described urethra catheter made of latex rubber has several drawbacks. Firstly, since a curing agent or the like is used, elution cannot be prevented, resulting in a problem in terms of safety when it is used. Therefore, it is not suitable to be kept for a long time in the body of a patient who has been subjected to an operation. Furthermore, since the balloon is machined by dip molding, its wall thickness cannot be rendered uniform causing a problem in terms of the machining facility.

Although elution can be reduced in the urethra catheter made of the above-described silicone rubber, a silicone type adhesive must be used for adhering the balloon to the shaft tube and adhering a cap for sealing the front end surface of the shaft tube. This deteriorates the machining facility. In addition, the elution from the adhesive taken place when the urethra catheter is kept in the body can deteriorate the safety when it is used.

FR-A-2371933 discloses a tracheal catheter comprising a tubular body and a balloon which can be made of the same or different material selected from natural rubber, silicon rubber, synthetic rubber, polyolefines, plasticized polyvinyl chloride and polyurethane. The balloon may be sealed to the body with or without an adhesive.

US-A-1428766 discloses an urethral or trachea catheter comprising a body and a balloon integrally moulded from plastics material and in particular polyvinyl chloride.

An object of the present invention is to provide a urethra catheter exhibiting a good safety when it is used and excellent manufacturing facility and a manufacturing method therefor.

An urethra catheter in accordance with the present invention is defined as set forth in claim 1.

Since the thermoplastic elastomer does not use a curing agent and must not use an adhesive due to its welding performance there is no risk of elution to be taken place when it is kept in the body, so that the urethra catheter can be used safely.

According to an embodiment of the present invention, the shaft tube and the balloon are respectively made of a material selected from said group.

When the shaft tube and the balloon inserted to the body are made of the thermoplastic elastomer, there is no risk of elution from the outer surface of the components which are brought into contact with the human body, so that the urethra catheter can be used safely.

According to another embodiment the shaft tube and the balloon are made of the same material selected from the said group, preferably of polystyrene thermoplastic elastomer.

When the shaft tube and the balloon to be inserted into the body are formed by the same type of thermoplastic elastomer, they can be easily welded to each other. Furthermore, the use of polystyrene thermoplastic elastomer permits to obtain excellent chemical resistance characteristics.

According to a further embodiment of the present invention, the shaft tube, the balloon, and the base portion are respectively made of at least a material selected from said group. Accordingly, except for a valve and a fixing rubber which are connected to the sub-lumen, and do not relate to the outer surface and the main lumen through which urine passes, the overall body is made of thermoplastic elastomer. Therefore, there is no risk of elution to take place in both the outer surfaces of the components which are brought into contact with the human body and the main lumen through which urine passes, so that the urethra catheter can be used extremely safely,

According to another embodiment, the shaft tube, the balloon, and the base portion are made of the same material selected from said group, preferably of polystyrene thermoplastic elastomer.

Accordingly, the overall body is made of the same thermoplastic elastomer. Therefore, welding of the components can be easily conducted since thay are made of the same material. When furthermore, the overall body is made of the polystyrene thermoplastic elastomer, excellent chemical resistance can be obtained.

In an embodiment of the present invention, there is provided an urethra catheter including a shaft tube having a main lumen and a sub-lumen and further having an urine inlet port at the front end thereof, said urine inlet port being connected to said main lumen; a balloon fastened on the outer surface of a front end portion of said shaft tube to cover an opening portion of said sub-lumen formed in said shaft tube; and a base portion disposed in the base end portion of said shaft tube, having an urine outlet port connected to said main lumen, and further having a fluid inlet/outlet port connected to said sub-lumen and capable of letting in or letting out fluid for expanding said balloon, said urethra catheter being characterized in that the front end portion of said shaft tube has a reduced diameter the balloon being welded to the shaft tube at said front end portion of reduced diameter so as to cover the outer surface of said front end portion withouty generating a step between said front end portion to which the balloon is fastened and the other portions, and said shaft tube and said balloon are made of a thermoplastic elastomer material selected from the group consisting of polystyrene, polyolefine, polyurethane, and vinyl thermoplastic elastomers.

A method of manufacturing a urethra catheter in accordance with the present invention comprises the steps of: manufacturing the shaft tube and the balloon; fastening the balloon to cover the outer portion of the front portion of the shaft tube, and then heating both end portions of the balloon, so as to weld them to the shaft tube; forming the base portion in the base end portion of the shaft tube; and machining the front portion of the shaft tube.

The components are respectively formed and assembled by a molding method utilizing the characteristics of the thermoplastic elastomer and by a welding method utilizing heat. Therefore, a urethra catheter exhibiting excellent dimension and appearance can be manufactured without the necessity of using an adhesive. Furthermore, excellent machining facility can be realized.

In an embodiment of the invention, the shaft tube is extrusion-molded, and cut so as to have a predetermined length; the front portion of the shaft tube is reduced in its diameter by the wall thickness of the balloon, and an opening portion connected to the sub-lumen is formed in the front end portion whose diameter has been reduced.

Accordingly, the front end portion of the shaft tube is reduced in its diameter (by a size corresponding to the thickness of the balloon) before the balloon is welded to the portion whose diameter has been reduced. Therefore, the diameter of the portion to which the balloon is fastened and the diameter of the other portions can be made the same, thus eliminating any supplemental step. As a result, when the urethra catheter is inserted into the body, the resistance to insertion is reduced.

The balloon may be manufactured by extrusion-molding or injection-molding.

Therefore, a balloon having a stable thickness can be provided.

Preferably, the balloon is welded to the shaft tube by bringing a heated mold into contact with both end portions of the balloon and rotating a wire core, so that the balloon is welded to the outer portion of the front end portion of the shaft tube and a wire core is inserted into the main lumen of the shaft tube.

Accordingly, the wire core inserted into the main lumen of the shaft tube is rotated on condition that both end portions of the balloon fastened to cover the outer surface of the front portion of the shaft tube are positioned in contact with the heated mold. Therefore, the balloon can be welded to the shaft tube securely and uniformly.

The base portion can be formed at the base end portion of the shaft tube by insert-molding in the shaft tube.

Since the base portion is insert-molded to the shaft tube, the base portion can thus be securely fastened.

In another embodiment, the base portion is formed at the base end portion of the shaft tube by injection molding of the base portion and welding said base portion with heat to the base end portion of the shaft tube, thus improving the working efficiency.

The front portion of the shaft tube is preferably machined in such a manner that a semispherical outer surface in which the main lumen and the sub-lumen are closed is formed by the manufacturing process in which an opening portion connected to the main lumen is formed in the portion more forward than the balloon of the shaft tube and by mold-machining the front end surface of the shaft tube with heat.

Since the front end surface of the shaft tube is molded with heat, the front end surface of the shaft tube can be finished uniformly and equally, and therefore, the front end surface can be sealed.

The balloon can be welded to the shaft tube in such a manner that the front end portion of the balloon to be welded to the shaft tube is arranged to have a larger size than another end portion of the balloon to be welded to the shaft tube, thus facilitating the machining of the front end of the shaft tube.

The invention will be better understood by the following description of an example illustrated by the annexed drawings on which :
FIG.1 is a view which illustrates the appearance of an urethra catheter according to an embodiment of the present invention :
FIG.2 is a cross sectional view which illustrate an essential portion of the urethra catheter shown in FIG.1;
FIG.3 is a cross sectional view which illustrates the balloon portion shown in FIG.1;
FIG.4 is a cross-sectional view which illustrates the shaft tube portion shown in FIG.1;
and FIG.5 is a flow chart which illustrates the method of manufacturing of the urethra catheter.

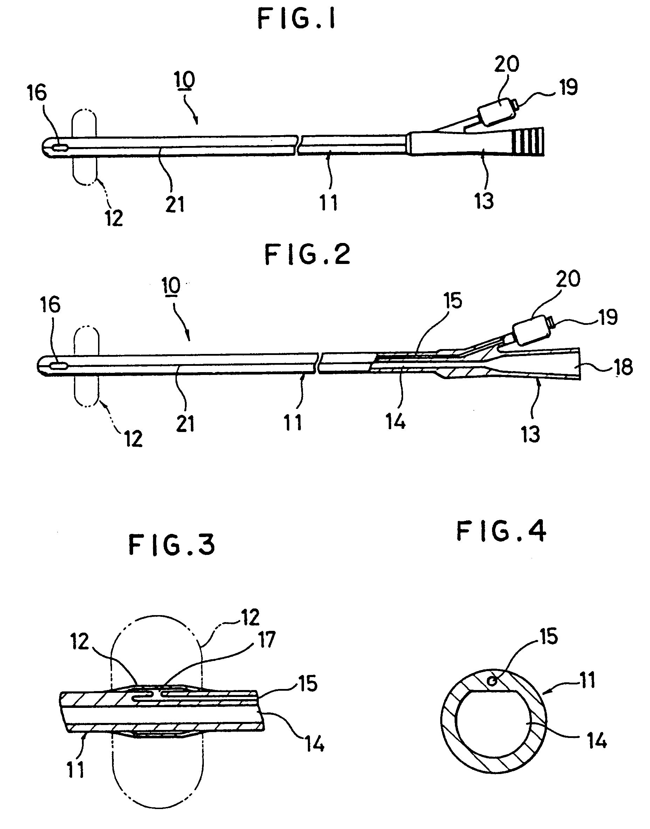

A urethra catheter 10 is, as shown on in FIGS.1 and 2, constituted by a shaft tube 11, a balloon 12, and a base portion 13.

As shown in FIGS.3 and 4, the shaft tube 11 has a main lumen 14, a sub-lumen 15, and a urine inlet port 16 connected to the main lumen 14, the urine inlet port 16 being formed in the side surface of the front portion thereof.

The balloon 12 is secured around an opening 17 on the outer surface of the front portion of the shaft tube 11, the opening 17 being formed in the sub-lumen 15 provided for the shaft tube 11.

A base portion 13 is disposed adjacent to the base end portion of the shaft tube 11 and has a urine outlet port 18 connected to the main lumen 14, the base portion 13 further having a valve 19 (fluid inlet/outlet port) connected to the sub-lumen 15 and capable of letting in or letting out fluid which acts to expand the balloon. The valve 19 is fastened to the end portion of the base portion 13, and the state of the fastening is fixed by a fixing rubber 20.

The urethra catheter 10 is used in such a manner that a urine outlet tube connected to a urine accumulating bag is connected to the urine outlet port 18. As a result, urine introduced through the urine inlet port 16 formed in the shaft tube 11 can be eliminated through the main lumen 14 and the urine outlet port 18. The balloon 12 is expanded when fluid for expanding the balloon such as sterilized water injected by a syringe inserted into the valve 19 is supplied through the sub-lumen 15.

The urethra catheter 10 has a contrast media line 21 formed on the surface of its shaft tube 11 in the direction of its axis.

The urethra catheter 10 can employ thermoplastic elastomers defined by the following groups (A) to (D) as the materials for its shaft tube 11, the balloon 12, and the base portion 13.
(A) Polystyrene thermoplastic elastomer
   For example, SEBS (Styrene-Ethylene-Butylene-Styrene), SBS (Styrene-Butadiene-Styrene), SIS (Styrene-Isoprene-Styrene)
   Preferable SEBS is exemplified by "RABALON" (trade name) manufactured by Mitsubishi Petro-Chemical Co., Ltd.
(B) Polyolefine thermoplastic elastomer
   For example, partially crosslinked polyolefine thermoplastic elastomer such as EPBM-polyethylene and butyl rubber-polypropylene
(C) Polyurethane thermoplastic elastomer
   For example, polyurethance, polyurethane-polyol
(D) Vinyl thermoplastic elastomer
   For example, vinyl acetate thermoplastic elastomer, polyvinyl chloride thermoplastic elastomer

In this case, the urethra catheter 10 is specifically constituted in a manner selected from the following manners (1) to (7).
(1) At least one of the shaft tube 11, the balloon 12, and the base portion 13 is made of a material selected from the above-described groups.
(2) The shaft tube 11 and the balloon 12 are respectively made of a material selected from the above-described groups.
(3) The shaft tube 11 and the balloon 12 are made of the same material selected from the above-described groups.
(4) The shaft tube 11 and the balloon 12 are made of the above-described polystyrene thermoplastic elastomer.
(5) The shaft tube 11, the balloon 12 and the base portion 13 are respectively made of at least a material selected from the above-described groups.
(6) The shaft tube 11, the balloon 12, and the base portion 13 are made of the same material selected from the above-described groups.
(7) The shaft tube 11, the balloon 12, and the base portion 13 are made of the above-described polystyrene thermoplastic elastomer.

A method of manufacturing the above-described urethra catheter 10 will now be described.

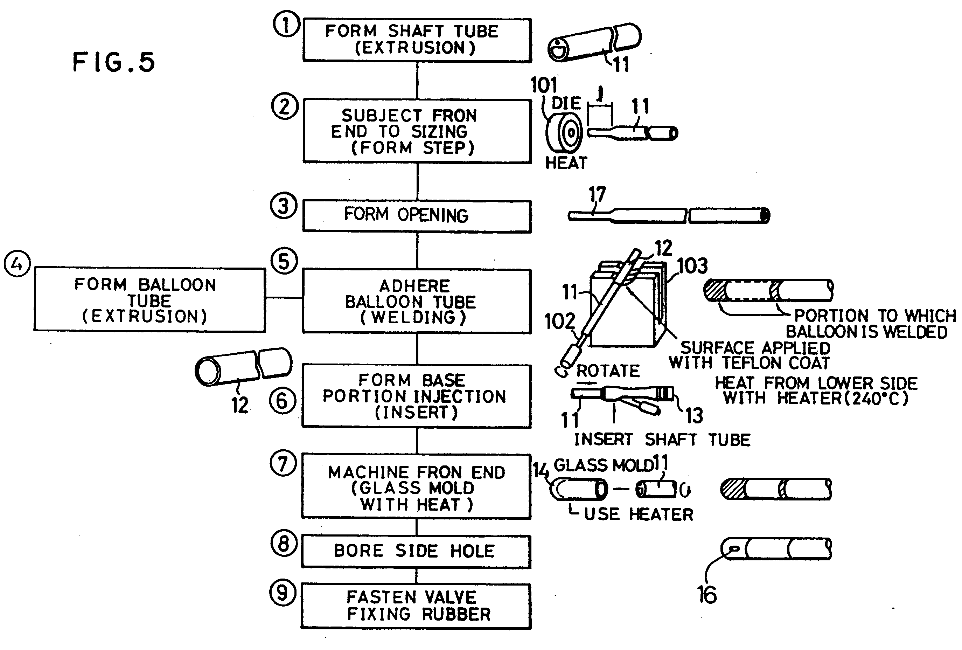

The urethra catheter 10 is manufactured by a method comprising steps selected from the manufacturing steps (1) to (6) illustrated on Fig. 5 :

Manufacturing Step (1) : The shaft tube 11 is manufactured (steps ①, ②, and ③).

This manufacturing step specifically consists of a first step (step ①) in which the shaft tube 11 is extrusion-molded, and the shaft tube 11 is cut so as to have a predetermined length (6, 8Fr must have a length of 280 mm, while 10 to 26Fr must have a length of 370 mm), a second step (step ②) in which the diameter of the front portion of the shaft tube 11 is reduced to have a predetermined diameter (ℓ) by using a heated sizing die 101, and a third step (step ③) in which the opening 17 connected to the above-described sub-lumen 15 is formed in the front end portion whose diameter has been reduced by the predetermined diameter.

The diameter obtained by the reduction must be arranged to be substantially the same as the wall thickness of the balloon 12 to be described later. As a result, the shaft tube 11 does not generate a step between the portion to which the balloon is fastened and the other portions.

Manufacturing Step (2) : The balloon 12 is manufactured (step ④)

In this manufacturing step the balloon 12 is extrusion-molded or injection-molded, and the balloon 12 thus molded is cut to have a predetermined length (15 to 21 mm).

It is preferable that the length obtained by cutting be similar to the predetermined diameter ℓ of the shaft tube 11 obtained by the diameter reduction.

The wall thickness of the balloon 12 is 0.4 mm.

Manufacturing Step (3) : The balloon 12 is disposed to cover the outer surface of the front portion of the shaft tube 11, and the two sides of the balloon 12 are heated so as to be welded to the shaft tube 11 (step ⑤).

In this manufacturing step the balloon 12 is caused to cover the outer surface of the front portion of the shaft tube 11 (it is preferable that the front portion is the front end portion whose diameter has been reduced by the predetermined diameter), and a wire core 102 is inserted into the main lumen 14 of the shaft tube 11. In this state, both end portions of the balloon 12 are brought into contact with the surfaces of recessed portions of a mold 103 whose both end portions are heated and made of, for example, brass before the wire core 102 is rotated. Thus, the two end portions of the balloon 12 are welded to the shaft tube 11 by an external heat application method (a method in which it is heated from outside by using a heater).

It is preferable that the front end portion of the portion to be welded be arranged to have a larger size in terms of easiness in conducting the machining of the front end portion to be described later.

The surfaces of the recessed portions of the mold 103 is subjected to Teflon® coating. The mold 103 is heated up to about 240°C by a heater.

The balloon 12 may be welded to the shaft tube 11 by an internal heat application method. The internal heat application method is a method in which a subject to be heated is placed in a coil to which high frequency current is supplied so as to cause the subject to generate heat. With this method, heating can be conducted uniformly.

Manufacturing Step (4) : In this step (step ⑥) the base portion 13 is formed at the base end portion of the shaft tube 11

In this manufacturing step the base portion 13 is molded so as to be the shaft tube 11 by insert molding.

In Another embodiment only the base portion 13 is previously manufactured, and this base portion 13 is welded by heat generated by high frequency welding to the base portion of the shaft tube 11.

Manufacturing Step (5) : In this manufacturing step (steps ⑦ and ⑧) the front portion of the shaft tube 11 is machined

Specifically, this step consists of first a manufacturing step (step ⑦) in which a slight quantity of silicone oil is applied to the front portion of the shaft tube 11, and this front portion is urged to be inserted into a cylindrical glass mold 104 (or a mold) having a bottom and whose front end portion has been heated, this front portion being inserted while rotated. As a result, the front portion is sealed so as to form a round shape by utilizing the thermoplasticity of the raw material. In a second manufacturing step (step ⑧) a urine inlet port 16 (side hole) connected to the main lumen 14 is formed in a portion which is positioned forward to the position of the balloon 12 of the shaft tube 11. As a result, a semispherical outer portion in which the main lumen 14 and the sub-lumen 15 are closed is formed at the front portion of the shaft tube 11.

Manufacturing Step (6) : In this manufacturing step (step ⑨) the valve 19 is fastened to the end portion of the base portion 13 and the fastening is fixed by a fixing rubber 20.

The urethra catheter 10 has the following advantages :
① In the case where the shaft tube 11, the balloon 12, and the base portion 13 is made of thermoplastic elastomer, the urethra catheter 10 can be used safely even if it is kept in the body because there is no fear of elution since no curing agent is used in the thermoplastic elastomer, and the same has good welding properties, thus eliminating the necessity of using an adhesive.
② In the case where the shaft tube 11 and the balloon 12 to be inserted into the body are made of thermoplastic elastomer, there is no fear of elution from the outer surface of the elements which are brought into contact with the human body. Therefore, the catheter can be used safely.
③ In the case where the shaft tube 11 and the balloon 12 to be inserted into the body are made of the same thermoplastic elastomer, they can be easily welded to each other since they are made of the same material in addition to the effect described in ②.
④ In the case where the shaft tube 11 and the balloon 12 to be inserted into the body are made of polystyrene thermoplastic elastomer, excellent chemical resistance is exhibited in addition to the effect described in ③.
⑤ In the case where the overall body of the shaft tube 11, the balloon 12, and the base portion 13 (except for the valve 19 and the fixing rubber 20. Since the valve 19 and the fixing rubber 20 are connected to the sub-lumen 15, they have no relationship with the outer surface and the main lumen 14 through which urine passes) are made of thermoplastic elastomer, there is no fear of elution from both the outer surface other than the portion of the components to be brought into contact with the human body and the main lumen 14 through which urine passes. Therefore, the urethra catheter 10 can be used extremely safely.
⑥ In the case where the overall body of the shaft tube 11, the balloon 12, and the base portion 13 are made of the same thermoplastic elastomer, they can be easily welded to each other since they are made of the same material in addition to the effect described shown in ⑤.
⑦ In the case where the overall body of the shaft tube 11, the balloon 12, and the base portion 13 are made of polystyrene thermoplastic elastomer, excellent chemical resistance is exhibited in addition to the effect described in ⑥.

The manufacturing method of the above-described urethra catheter 10 has the following advantages:
⑧ Since each of the components is formed and assembled with a molding method utilizing the characteristics of the thermoplastic elastomer and a heat welding method, the urethra catheter 10 exhibiting good dimension and appearance can be manufactured without use of an adhesive, and a satisfactory machining facility can be obtained
⑨ The diameter of the front portion of the shaft tube 11 is reduced (by a size corresponding to the thickness of the balloon 12) so as to weld the balloon 12 to the thus size-reduced portion. As a result, the diameter of the portion to which the balloon 12 is fastened and the diameter of the other portion can be made the same, thus eliminating any step. Therefore, resistance caused during insertion can be reduced.
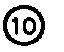 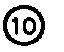 Since the balloon 12 is manufactured by extrusion molding or injection molding, the balloon 12 can have a stable thickness
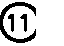 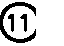 Since the wire core 102 inserted into the main lumen 14 of the shaft tube 11 is rotated in a state where both end portions of the balloon 12 fastened to cover the outer surface of the front portion of the shaft tube 11 are positioned to contact with the heating mold 103, the balloon 12 can be welded to the shaft tube 11 in a secure and uniform manner.
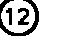 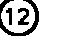 Since the base portion 13 is insert-molded to the shaft tube 11, the fastening of the base portion 13 can be conducted securely
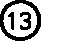 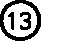 Since the base portion 13 manufactured by injection molding is welded to the base end portion of the shaft tube 11 by utilizing heat, the working efficiency can be improved.
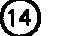 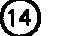 Since the front end portion of the shaft tube 11 is molded by the glass mold 104 with heat applied thereto, the surface of the front portion of the shaft tube 11 can be finished uniformly and equally so that this front end surface can be sealed.
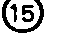 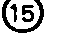 Since the front end portion of the balloon 12 to be welded to the shaft tube 11 is arranged to have a larger size than another end portion of the balloon 12 to be welded to the shaft tube 11, the machining of the front end portion of the shaft is made easily.

Table 1 shows the results of experiments carried out to evaluate elution taken place in each of the urethra catheters according to the present invention and comparative examples.

Symbol (A) according to the present invention represents a sample employing polystyrene thermoplastic elastomer as its material, while Embodiments 1, 2, and 3 were made of SEBS, SBS, and SIS, respectively.

Symbol (B) according to the present invention represents a sample employing polyolefine elastomer as its material, while Embodiments 4 and 5 were made of partially crosslinked EPDM-polyethylene and butyl rubber-polypropylene, respectively.

Symbol (C) according to the present invention represents a sample employing polyurethane thermoplastic elastomer as its material, while Embodiments 6 and 7 were made of polyurethane-polyester and polyurethane-polyol, respectively.

Symbol (D) according to the present invention represents a sample employing vinyl thermoplastic elastomer as its material, while Embodiments 8 and 9 here made of ethylene-vinyl acetate and polyvinyl chloride elastomers, respectively.

The test methods for evaluating ΔKMnO₄ and ΔPH factors were conducted in accordance with rubber test method with a disposable blood transfusion set and fluid transfusion method. The test method for evaluating UV absorbancy was conducted in such a manner that absorbance was measured under conditions that the layer length gas 10 mm and wavelength gas 220 nm by using test fluid according to the above-described rubber test method and novel test fluid serving as a reference. The factor of UV absorbance was conducted so as to measure the absorbancy of ultraviolet rays (220 nm) displayed by the test fluid. It means that the smaller the absorbance becomes, the more the portion in which the elution occurs can be reduced.

As is shown from Table 1, according to the present invention, the quantity of elution can be reduced satisfactorily with respect to the case in which silicone rubber is employed. Safety in use can thus be obtained.

As described above, according to the present invention, an urethra catheter exhibiting excellent safety when it is used and satisfactory manufacturing easiness can be obtained.

**Table 1**

| | | | ΔKMnO₄ mℓ | ΔPH | UV Absorbance 220 nm Abs |
|---|---|---|---|---|---|
| Examples of the present invention | A | Embodiment 1 | 0.02 | 0.21 | 0.004 |
| | | Embodiment 2 | 0.03 | 0.20 | 0.004 |
| | | Embodiment 3 | 0.02 | 0.21 | 0.005 |
| | B | Embodiment 4 | 0.02 | 0.20 | 0.004 |
| | | Embodiment 5 | 0.04 | 0.21 | 0.004 |
| | C | Embodiment 6 | 0.03 | 0.23 | 0.003 |
| | | Embodiment 7 | 0.03 | 0.22 | 0.003 |
| | D | Embodiment 8 | 0.02 | 0.21 | 0.005 |
| | | Embodiment 9 | 0.03 | 0.22 | 0.003 |
| Comparative Example 1 | | | 0.50 | 0.29 | 0.292 |
| Comparative Example 2 | | | 0.10 | 0.21 | 0.005 |
| Test Method | | | According to rubber test method based on disposable blood transfusion set and fluid transfusion set | | Absorbance of the test fluid according to the ΔKMnO₄ and ΔPH tests and the same of test fluid which had not been subjected to any test were measured under condition that the layer length was 10mm and the wavelength 220nm |

## Claims

1. An urethra catheter including a shaft tube (11) having a main lumen and a sub-lumen and further having an urine inlet port at the front end thereof, said urine inlet port being connected to said main lumen; a balloon (12) fastened on the outer surface of a front end portion of said shaft tube to cover an opening portion of said sub-lumen formed in said shaft tube; and a base portion (13) disposed in the base end portion of said shaft tube, having an urine outlet port connected to said main lumen, and further having a fluid inlet/outlet port connected to said sub-lumen and capable of letting in or letting out fluid for expanding sad balloon, said shaft tube (11) and said balloon (12) being made of a thermoplastic elastomer material selected from the group consisting of polystyrene, polyolefine, polyurethane, and vinyl thermoplastic elastomers, said urethra catheter being characterized in that the front end portion of said shaft tube has a reduced diameter, the balloon (13) being welded to the shaft tube at said front end portion of reduced diameter so as to cover the outer surface of said front end portion without generating a step between said front end portion to which the balloon is fastened and the other portions.

2. An urethra catheter according to claim 1, wherein said shaft tube and said balloon are made of the same material.

3. An urethra catheter according to claim 2, wherein said shaft tube and said balloon are made of polystyrene thermoplastic elastomer.

4. An urethra catheter according to anyone of claims 1 to 3, wherein said base portion is also made of a material selected from said group.

5. An urethra catheter according to claim 4, wherein said shaft tube, said balloon, and said base portion are made of the same material.

6. An urethra catheter according to claim 5, wherein said shaft tube, said balloon, and said base portion are polystyrene thermoplastic elastomers.

7. A method of manufacturing an urethra catheter according to claim 1 comprising:
- extrusion-molding and cutting the shaft tube so as to have a predetermined length:
- reducing the diameter of a front end portion of said shaft tube by the wall thickness of the balloon;
- forming an opening portion connected to said sub-lumen in said front end portion whose diameter has been reduced.
- extrusion-molding or injection-molding said balloon;
- welding said balloon to said shaft tube by bringing into contact a heated mold with said both end portions of said balloon and rotating a wire core, so that said balloon is welded to said outer surface of said front end portion of said shaft tube and said wire core is inserted into said main lumen of said shaft tube;
- forming said base portion in the base end portion of said shaft tube;
- machining said front portion of said shaft tube to form a semispherical outer surface in which said main lumen and said sub-lumen are closed; and
- forming an opening portion connected to said main lumen in the portion more forward than the balloon of said shaft tube by mold-machining the front end surface of said shaft tube with heat.

8. A method of manufacturing an urethra catheter according to claim 7, wherein said base portion is formed at said base end portion of said shaft tube by forming said base portion by insert-molding in said shaft tube.

9. A method of manufacturing an urethra catheter according to claim 7, wherein said base portion is formed at said base end portion of said shaft tube by manufacturing said base portion by injection molding and welding with heat to said base end portion of said shaft tube.

10. A method of manufacturing an urethra catheter according to claim 7, wherein said balloon is welded to said shaft tube, said front end portion of said balloon to said shaft tube having a larger size than another end portion of said balloon to be welded to said shaft tube.

## Patentansprüche

1. Harnröhrenkatheter, umfassend ein(en) Schaftrohr oder -schlauch (11) mit einem Hauptlumen und einem Nebenlumen sowie einem an seinem Vorderende vorgesehenen, mit dem Hauptlumen verbundenen Harneinlaß; einen an der Außenfläche eines Vorderabschnitts des Schaftschlauches, einen im Schaftschlauch geformten Öffnungsteil des Nebenlumens bedeckend angebrachten Ballon (12); und einen im bzw. am Basisendabschnitt des Schaftschlauches angeordneten Basisabschnitt (13) mit einem mit dem Hauptlumen verbundenen Harnauslaß sowie einem Fluideinlaß/auslaß, der mit dem Nebenlumen verbunden und über den ein Fluid zum Aufblasen oder Aufweiten des Ballons einführbar oder abführbar ist, wobei der Schaftschlauch (11) und der Ballon (12) aus einem thermoplastischen elastomeren Werkstoff aus der Gruppe thermoplastische Polystyrol-, Polyolefin-, Polyurethan- und Vinyl-Elastomere geformt sind, wobei der Harnröhrenkatheter dadurch gekennzeichnet ist, daß der Vorderendabschnitt des Schaftschlauches einen verringerten Durchmesser aufweist und der Ballon (13 bzw. 12) am Vorderendabschnitt verringerten Durchmessers am Schaftschlauch, die Außenfläche des Vorderendabschnitts bedeckend, angeschweißt ist, ohne eine Stufre zwischen dem Vorderendabschnitt, an welchem der Ballon befestigt ist, und den anderen Abschnitten zu bilden.

2. Harnröhrenkatheter nach Anspruch 1, wobei der Schaftschlauch und der Ballon aus dem gleichen Werkstoff geformt sind.

3. Harnröhrenkatheter nach Anspruch 2, wobei der Schaftschlauch und der Ballon aus einem thermoplastischen Polystyrolelastomer geformt sind.

4. Harnröhrenkatheter nach einem der Ansprüche 1 bis 3, wobei der Basisabschnitt ebenfalls aus einem Werkstoff aus der genannten Gruppe geformt ist.

5. Harnröhrenkatheter nach Anspruch 4, wobei der Schaftschlauch, der Ballon und der Basisabschnitt aus dem gleichen Werkstoff geformt sind.

6. Harnröhrenkatheter nach Anspruch 5, wobei der Schaftschlauch, der Ballon und der Basisabschnitt aus thermoplastischen Polystyrolelastomeren bestehen.

7. Verfahren zur Herstellung eines Harnröhrenkatheters nach Anspruch 1, umfassend die Schritte:
- Extrusions- bzw. Strangpreßformen des Schaftschlauches und Schneiden desselben auf eine vorbestimmte Länge,
- Verkleinern des Durchmessers eines Vorderendabschnitts des Schaftschlauches um die Wanddicke des Ballons,
- Formen eines mit dem Nebenlumen verbundenen Öffnungsteils im Vorderendabschnitt, dessen Durchmesser verkleinert worden ist,
- Strangpreß- oder Spritzgießformen des Ballons,
- Anschweißen des Ballons am Schaftschlauch durch Inberührungbringen eines erwärmten Formteils mit beiden Endabschnitten des Ballons und Drehen eines Drahtkerns, so daß der Ballon an der Außenfläche des Vorderendabschnitts des Schaftschlauches angeschweißt wird und der Drahtkern (dabei) in das Hauptlumen des Schaftschlauches eingeführt ist,
- Formen des Basisabschnitts im (am) Basisendabschnitt des Schaftschlauches,
- (maschinelles) Verformen (machining) des Vorderendabschnitts des Schaftschlauches zum Anformen einer halbkugeligen Außenfläche, in welcher Haupt- und Nebenlumen verschlossen sind, und
- Ausbilden eines mit dem Hauptlumen verbundenen Öffnungsteils in dem vorderhalb des Ballons gelegenen Abschnitt des Schaftschlauches durch (maschinelles) Verformen der Vorderend- oder Stirnfläche des Schaftschlauches mittels Wärme.

8. Verfahren zur Herstellung eines Harnröhrenkatheters nach Anspruch 7, wobei der Basisabschnitt am Basisendabschnitt des Schaftschlauches durch Anformen des Basisabschnitts durch Einsatzformen oder Umspritzen im (am) Schaftschlauch geformt wird.

9. Verfahren zur Herstellung eines Harnröhrenkatheters nach Anspruch 7, wobei der Basisabschnitt am Basisendabschnitt des Schaftschlauches durch Herstellung des Basisabschnitts durch Spritzgießen oder -formen und thermisches Anschweißen am Basisendabschnitt des Schaftschlauches geformt wird.

10. Verfahren zur Herstellung eines Harnröhrenkatheters nach Anspruch 7, wobei der Ballon am Schaftschlauch angeschweißt wird und wobei der Vorderendabschnitt des Ballons zum oder am Schaftschlauch ein größeres Maß aufweist als ein anderer Endabschnitt des am Schaftschlauch anzuschweißenden Ballons.

## Revendications

1. Cathéter urétral comprenant une tige tubulaire (11) comportant une lumière principale et une lumière secondaire et comportant, en outre, un orifice d'entrée d'urine à son extrémité avant, ledit orifice d'entrée d'urine étant raccordé à ladite lumière principale; un ballonnet (12) fixé à la surface extérieure d'une partie d'extrémité avant de ladite tige tubulaire pour recouvrir une partie ouverture de ladite lumière secondaire formée dans ladite tige tubulaire; et une partie base (13) disposée dans la partie d'extrémité de base de ladite tige tubulaire, comportant un orifice de sortie d'urine raccordé à ladite lumière principale, et comportant, en outre, un orifice d'entrée/sortie de fluide raccordé à ladite lumière secondaire et apte à laisser entrer et à laisser sortir le fluide pour dilater ledit ballonnet, ladite tige tubulaire (11) et ledit ballonnet (12) étant formés d'une matière élastomère thermoplastique choisie parmi le groupe comprenant le polystyrène, la polyoléfine, le polyuréthane, et les élastomères thermoplastiques vinyliques, ledit cathéter urétral étant caractérisé en ce que la partie d'extrémité avant de la tige tubulaire a un diamètre réduit, le ballonnet (13) étant soudé à la tige tubulaire au niveau de ladite partie d'extrémité avant de diamètre réduit, de manière à recouvrir la surface extérieure de ladite partie d'extrémité avant sans créer un épaulement entre ladite partie d'extrémité avant à laquelle est fixé le ballonnet et les autres parties.

2. Cathéter urétral selon la revendication 1, dans lequel ladite tige tubulaire et ledit ballonnet sont formés du même matériau.

3. Cathéter urétral selon la revendication 2, dans lequel ladite tige tubulaire et ledit ballonnet sont formés d'un élastomère thermoplastique à base de polystyrène.

4. Cathéter urétral selon l'une quelconque des revendications 1 à 3 , dans lequel ladite partie base est également formée d'un matériau choisi parmi ledit groupe.

5. Cathéter urétral selon la revendication 4, dan lequel ladite tige tubulaire, ledit ballonnet, et ladite partie base sont formés du même matériau.

6. Cathéter urétral selon la revendication 5, dans lequel ladite tige tubulaire, ledit ballonnet et ladite partie base sont formés d'élastomères thermoplastiques à base de polystyrène.

7. Procédé de fabrication d'un cathéter urétral selon la revendication 1, comprenant :
le moulage par extrusion et la coupe de la tige tubulaire à une longueur prédéterminée;
la réduction du diamètre d'une partie d'extrémité avant de ladite tige tubulaire à l'épaisseur de paroi du ballonnet;
la formation d'une partie ouverture raccordée à la dite lumière secondaire dans ladite partie d'extrémité avant dont le diamètre a été réduit;
le moulage par extrusion ou le moulage par injection dudit ballonnet;
le soudage dudit ballonnet à ladite tige tubulaire par mise en contact d'un moule chauffé avec les deux parties d'extrémité précitées dudit ballonnet et la rotation d'un noyau formé par un fil métallique de manière que ledit ballonnet soit soudé à ladite surface extérieure de la partie d'extrémité avant de ladite tige tubulaire et que ledit noyau formé par un fil métallique soit inséré dans la lumière principale de ladite tige tubulaire;
la formation de ladite partie base dans ladite partie d'extrémité de base de la tige tubulaire;
l'usinage de ladite partie avant de la tige tubulaire pour former une surface extérieure hémisphérique dans laquelle sont fermées ladite lumière principale et ladite lumière secondaire; et
la formation d'une partie ouverture raccordée à ladite lumière principale dans la partie, située plus en avant que le ballonnet, de la tige tubulaire par usinage-moulage de la surface d'extrémité avant de la tige tubulaire au moyen de chaleur.

8. Procédé de fabrication d'un cathéter urétral selon la revendication 7, dans lequel on forme ladite partie base au niveau de ladite partie d'extrémité de base de la tige tubulaire en formant ladite partie base par surmoulage dans ladite tige tubulaire.

9. Procédé de fabrication d'un cathéter urétral selon la revendication 7, dans lequel on forme ladite partie base au niveau de ladite partie d'extrémité de base de la tige tubulaire en fabriquant ladite partie base par moulage par injection et par soudage, à l'aide de chaleur, à ladite partie d'extrémité de base de la tige tubulaire.

10. Procédé de fabrication d'un cathéter urétral selon la revendication 7, dans lequel on soude ledit ballonnet à ladite tige tubulaire, ladite partie d'extrémité avant dudit ballonnet jusqu'à ladite tige tubulaire ayant une dimension plus grande qu'une autre partie d'extrémité dudit ballonnet devant être soudée à ladite tige tubulaire.
